# EUROPEAN PATENT SPECIFICATION

(11) **EP 0 876 304 B1**
(45) Date of publication and mention of the grant of the patent: **27.06.2001**
(21) Application number: 96929826.4
(22) Date of filing: 28.08.1996
(51) Int. Cl.: C02F 3/34, C02F 3/00

(54) **SOIL AND WATER REMEDIATION AND ENHANCEMENT PROCESS AND APPARATUS THEREFOR**
VERFAHREN UND VORRICHTUNG ZUR ENTGIFTUNG VON ERDBODEN UND WASSER
AMELIORATION DU SOL ET DE L'EAU ET PROCEDE ET APPAREIL CORRESPONDANTS

(30) Priority: 29.08.1995 US 520826
(43) Date of publication of application: 11.11.1998
(73) Proprietor: Eco Soil Systems, Inc., San Diego, CA 92127 (US)
(72) Inventor: RUNYON, Larry, LAS CRUCES NEW MEXICO 88005 (US)
(74) Representative: Croston, David
(86) International application number: PCT/US96/14008
(87) International publication number: WO 97/08106

(56) References cited:
- EP-A- 0 228 626
- DE-A- 1 920 328
- US-A- 5 227 067
- US-A- 5 227 068
- DATABASE WPI Section Ch, Week 9521 Derwent Publications Ltd., London, GB; AN 95160038 XP002021240 & RU,A,2 019 527 (SEV GNI I PK GEOL TS) , 15 September 1994

## Description

### BACKGROUND OF THE INVENTION

### Field of the Invention:

The invention herein relates to processes for improving the conditions of soil and water and to improve their ability to support vegetation, including crops. More particularly, it relates to such methods which use microorganisms to enhance the soil and water properties.

### Background of the Invention:

Improvement of soil and water conditions to enhance vegetation growth is a subject of significant interest and importance. Parks, golf courses, cemeteries, sod farms, athletic fields, and similar locations all need extensive decorative and functional vegetation, including grass, shrubbery and trees. The vegetation must be easy to maintain, provide pleasing visual appearance and be hardy, to sustain itself throughout extensive use, particularly for areas such as parks, athletic fields and golf courses. Commercial horticulture, such as greenhouses and production of landscape and bedding plants, also requires soil and water which are supportive of vigorous and healthy growth of the commercial plants. Similarly, agriculture also requires soil and water conditions which support optimum plant growth, whether of field crops, row crops or tree crops.

Many water supplies or water bodies are contaminated in one manner or another. It is typical for well water to include contaminants which were originally dissolved or entrained in ground water which subsequently flowed into the wells, carrying the contaminants with it. At the other end of the scale, waste water collection ponds, which of course are highly contaminated at the outset, must often be treated to reduce the contamination so that the water can be reused for various purposes and so that high degrees of residual contamination do not accumulate in a waste water pond over a period of time.

Several previous patents have issued which have dealt to some extent with these various issue. These patents (Nos. 5,227,067; 5,227,068; and 5,314,619, all in the name of L. Runyon, and assigned to Eco Soil Systems, Inc. of San Diego, California) deal with a variety of aspects of soil remediation and enhancement and water treatment by applying various microorganisms, enzymes and nutrients for the microorganisms to soil and water. The systems described in those patents have proved to be quite successful, and substantial benefits have been obtained for such application areas as golf courses, parks and field crops. However, these previous systems utilized solid reactants which had to be dissolved or dispersed prior to use, or were cumbersome and not particularly suited for soil or water treatment over large areas or in a wide variety of different types of applications. In particular, the handling of the solid reactants often posed problems with respect to different dissolution rates, concentrations and growth rates.

### SUMMARY OF THE INVENTION

The invention herein provides a convenient system which, while retaining all of the beneficial aspects of the previous systems, is substantially simplified with respect to handling growth and dispersion of micro-organisms in solution and is also adaptable to many different end-use applications, including treatment of turf, ornamental vegetation, horticultural and agricultural crops. Provision of the initial micro-organism materials in the form of aqueous suspensions which are incorporated into a large volume of water in a vessel and subsequent growth of biomass in the vessel provides for simplicity and flexibility not previously obtained from prior art devices and methods.

Therefore, in one embodiment the present invention provides a method for remediation and enhancement of soil or water, the method comprising the steps of:
forming a concentrated aqueous suspension of micro-organisms and nutrients therefor together in a first vessel;
providing a plurality of such suspensions in a plurality of such vessels.,
injecting said aqueous suspensions as batches into a substantially larger volume of water in a common second vessel;
retaining said larger volume of water with said suspension dispersed therein in said second vessel at a temperature and for a time sufficient for said micro-organisms to feed on at least a portion of said nutrients, reproduce and multiply into a concentrated biomass containing a remainder of said nutrients and an increased number of said micro-organisms in said water;
thereafter dispensing said biomass from said second vessel and dispersing said biomass on soil or water; and
maintaining said micro-organisms alive and active with said remainder of said nutrients for a period of time sufficient to enhance predetermined desirable properties of said soil or water or reduce predetermined undesirable properties of said soil or water.

If the biomass is dispensed continuously, aqueous suspensions of nutrients will be continuously added to the vessel to maintain the biomass density.

In yet another embodiment, the invention provides apparatus for remediation and enhancement of soil or water which comprises:
a plurality of first vessels for receiving concentrated aqueous suspensions of micro-organisms and nutrients therefor;
a common second vessel larger than each of said first vessels;
said first vessels being provided within said second vessel;
a liquid conduit between said second vessel and said plurality of first vessels;
an injector for moving said aqueous suspensions into a volume of water substantially greater than the volume of one of said first vessels and within said second vessel;
operating means for producing and maintaining conditions over a period of time within said second vessel conducive to reproduction and growth of said micro-organisms in the presence of said nutrients, such that said micro-organisms during said period of time multiply into an aqueous biomass comprising said micro-organisms dispersed in the vessel water; and
dispensing means for removing aqueous biomass from said second vessel;
such that said aqueous biomass can subsequently be dispersed into soil or water and said micro-organisms therein maintained alive and active for a period of time sufficient to enhance predetermined desirable properties of said soil or water or reduce predetermined undesirable properties of said soil or water.

Micro-organisms useful herein will enhance plant growth, provide pest suppression or eradication, soil or water detoxification, solids degradation or any combination thereof, exemplary micro-organisms include thermophiles, micro-organisms which utilise hydrocarbons as growth substrates, nitrogen fixing bacteria, halophiles, oxygen-generating bacteria, specific disease control agents, broad spectrum disease control agents, micro-organisms for thatch or rubble degradation, and micro-organisms which function as insecticides, fungicides, metabolites and/or herbicides. The system also functions to encourage microbial growth to stimulate metabolite production, such as, of antibiotics, that perform as fungistats, bactericides and the like.

### BRIEF DESCRIPTION OF THE DRAWINGS

Figure 1 is a schematic diagram of the basic process of the present invention, illustrating liquid feeders, biomass growth reactors and control systems for controlling the operation of the process.

Figures 2, 3 and 4 are schematic diagrams of different systems for dispersing the biomasses grown in the present process, including dispersion by spray and irrigation, dispersion remotely by vehicles, and dispersion remotely by connected systems.

Figures 5, 6 and 7 illustrate pictorially different end-use applications of the present process, including the treatment of turf and ornamental plants (illustrated as a golf course in Figure 5), use in horticulture (illustrated by treatment of greenhouse plants in Figure 6), and use in agriculture (illustrated by treatment of row crops in Figure 7).

Figures 8 and 9 illustrate schematically respectively, the treatment of well water by the process of this invention for purification of the water and charging the water with nutrients and treatment of waste water (Figure 9), illustrating the use of the process for reduction of solids and toxins and purification for ultimate reuse of the cleaned up affluent.

Figure 10 illustrates a multi-chambered biomass growth reactor which provides for isolated growth different microbes.

Figure 11 illustrates a biomass growth reactor in which indiscriminate growth microorganisms, such as fungi, can be grown and then the resulting biomass is macerated to permit free flow of the microorganisms through the system.

Figure 12, divided into sections A and B, illustrate two means of delivery of the compositions herein: by airplane (12-A) and manually operated distributor (12-B).

Figure 13 illustrates schematically the use of the invention for the purpose of ground water remediation.

### DETAILED DESCRIPTION AND PREFERRED EMBODIMENTS

The process of this invention will be best understood by reference to the drawings.

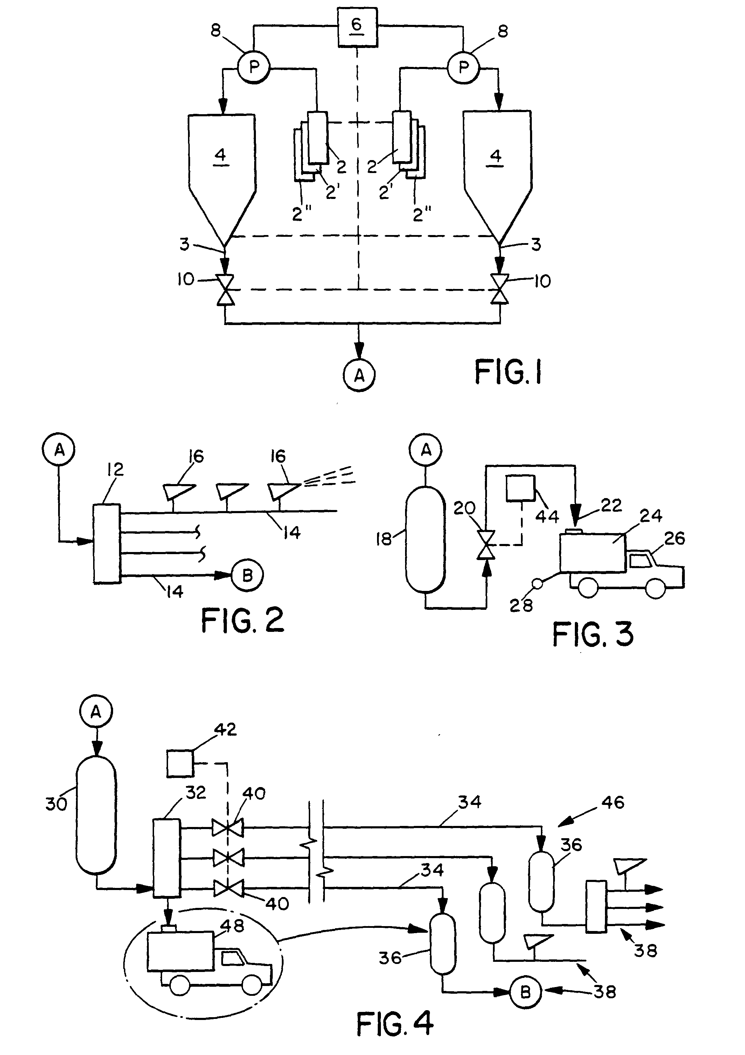

In Figure 1, the basic process is shown. A critical aspect is initially providing concentrated liquid solutions of microorganisms, enzymes and nutrients through feed devices 2. The use of the liquid microorganism feed from the concentrated containers 2 substantially enhances the present process with respect to the prior art which utilized solid initial reactants which had to be incorporated directly into the biomass growth reactors. Use of liquid feed through separate concentrate feed tanks 2 allows the growth process for the biomass in the growth reactors 4 to proceed much more rapidly at the start and with much greater uniformity, since the liquid microorganism feeds are readily dispersed throughout the growth tanks 4, rather then being concentrated at or near a solid feed container immersed in the liquid in the growth tank. A rapid and effective dispersion of the liquid throughout the growth tank allows for optimum biomass growth throughout the tank, rather than having some areas, close to a solid feed container, having overstimulated growth and other areas, at some distance from the solid container, being essentially starved for microorganisms.

The various microorganisms (which will be illustrated specifically below) may either be mixed in liquid form in the concentrate feed containers 2, or individual microorganisms or mixtures of microorganisms may be contained in a plurality of feed concentrate containers (designated 2, 2' and 2"). Use of a plurality of concentrate containers 2 may be advantageous where there are a wide variety of microorganisms which are intended to be used and where different microorganisms are to be injected into the system at different times, or in different concentrations. Similarly, it is useful where a much larger quantity of one or a few types of microorganisms are to be used as compared to others, since the ones that need to be replenished frequently can be isolated in their own container or containers 2 which can be readily and frequently restocked or replaced, while the microorganisms, enzymes and nutrients which are used at a much slower rate can be kept in their own original concentrate containers.

The system is controlled by controller 6 which is preferably a microprocessor of appropriate type. The controller 6 senses the concentration and other operating conditions of the containers 2 and reactors 4, as indicated by the dashed lines, and at the preprogrammed times will stop or start pumps 8 to inject the initial concentrated liquid microorganism slurries or concentrated liquid nutrients from the tanks into the reactors 4, or will open valves 10 either to drain the reactors 4 completely of the accumulated biomass after the growth cycle is completed or to continuously drain the reactors 4 during a continuous flow cycle. Commercial tanks, sensors, controllers, and pumps and valves are all readily available. Some are described in the patents cited previously and others are described widely in the literature and commercial sources, such as catalogs. Those skilled in the art will be well aware of the various types of equipment which are available and appropriate herein.

The system in Figure 1 is shown as having two parallel sets of concentrate tanks 2 and reactor 4. In some cases the process can be operated with a single reactor 4 and appropriate associated concentrate tank or tanks 2. However, this has the disadvantage that when a cycle is completed, the entire system must be shut down so that new supplies of the concentrated microorganisms, enzymes and nutrients can be supplied either to concentrate tank 2 or new tanks 2 inserted. More importantly however, the reactor 4 must be shut down for cleaning and sterilization and sanitizing. While a single system is shut down of course, there is no biomass being provided to the end use application, whether that is agriculture, horticulture or other application. It is therefore preferred to have at least two parallel systems, as illustrated in Figure 1, each operating at a different point in the cycle of vessel cleaning, sterilization and sanitation; water addition; injection of the liquid feed suspension; growth of the biomass; and draining of the vessel and dispensing of the biomass, so that when one of the units is either shut down for maintenance and recharging, awaiting the slow growth of certain types of microorganisms, or in the event of some sort of malfunction, the other system can be operating to be producing the biomass and dispersing it to the end use application. In this regard it is contemplated that larger commercial systems utilizing the present invention may contain three or more parallel reactor/liquid feed concentrate units so that one or more may be operating at any given time, even while others are shut down for maintenance or are in a portion of the biomass growth cycle where it is not appropriate to disperse any of the biomass slurry. Those skilled in the art will be able to determine the optimum number of units for any given end use, depending on factors such as the area of soil or volume of water to be treated, the type and concentration of vegetation involved, and the cost of equipment.

Alternatively to operating two, three or more reactor/liquid feed concentrate units in parallel, a single unit may be run in a continuous flow cycle. The process of inoculating the reactor 4 from the liquid feed tanks 2 is as described above; however, once the biomass in reactor 4 has reached a sustainable density, it is drained through valve 10 at a constant rate which is consonant with maintenance of the biomass through continuous feeding from tanks 2. Thus, the soil or water being treated (or manifold(s), etc. being filled) may be continuously provided with a flow of biomass while the biomass within the reactor 4 is provided with sufficient nourishment to continue growing, thereby replenishing what is used. One advantage to this continuous process of feeding and dispersing the biomass is that the reactor 4 need not be cleaned and sterilized after each growth cycle. Those of skill in the art will be able to determine the rates of removal of the biomass and addition of nutrients to the reactor to maintain the continuous fermentation of the biomass.

The tanks 2 and reactors 4 may be of any convenient size. It has been found particularly effective to have them sized such that an entire system containing a controller 6, one or more concentrate tanks 2 and the reactor 4 can be contained on a single pallet which can be moved by a forklift vehicle or contained on a trailer which is integral to or can be towed by a truck or similar vehicle from location to location. Such a trailer may be open or closed. A closed trailer of course, allows a system to operate while being sheltered from weather, dust and dirt. The connection A to the various end use applications would be mounted on the outside of the cover of the closed trailer so that connection by hose coupling or other conventional means could be easily obtained. Similarly, on skid or pallet mounted units, the coupling A will also be mounted on the pallet or skid, again for ease in coupling to other end use application systems. Of course, in many cases the units of the present invention will be permanently mounted at a central location, such as for a golf course or a park or athletic field, and a large plurality of individual systems can be placed together or a fewer number of systems with larger reactors and related equipment can be used.

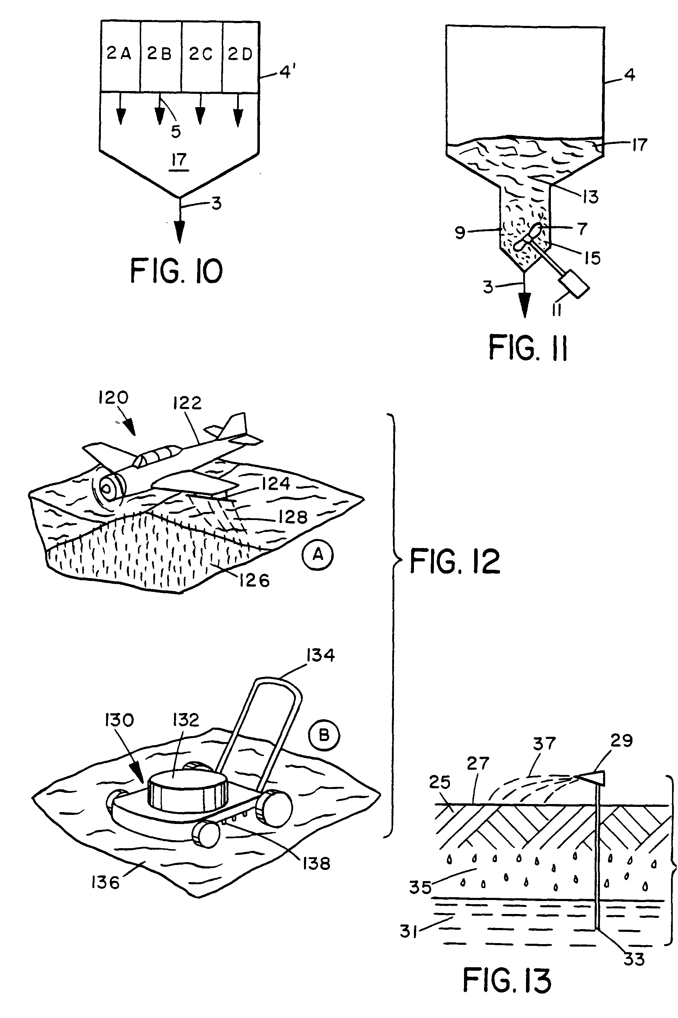

Figure 10 illustrates an alternative configuration of a biomass reactor 4'. This reactor includes a plurality of chambers illustrated as 2A, 2B, 2C and 2D, each of which can be used for isolated growth of individual microorganism biomasses or for storage of concentrated nutrient suspensions. At an intermediate stage or upon completion of growth one or more of these biomasses or nutrient solutions can then be dispensed into the mass of water slurry in common chamber 17, in which further mixing or growth can take place, prior to draining of the reactor 4' into the system through pipe 3.

Yet another embodiment of the reactor 4 is shown in Figure 11, for use when the biomass is or includes a microorganism with indeterminate growth, such as a fungus. The resulting biomass 13 in chamber 17 is tangled and not easily flowable. Therefore the reactor 4 is modified by addition of a maceration section 9 in which there is a macerator 7 (essentially a chopping blade mechanism), driven by motor 11, which is used to chop the biomass into finely divided pieces 15 which can be suspended in the water and flow easily through the outlet pipe 3 and on through the system. The ability to use fungi, and particularly fungal spores, in the system enables delivery of viable, active and infective microorganisms to the target site.

Typical and important end means of dispersing the biomass grown in the reactors 4 are illustrated in Figures 2-4. In each case, the interconnection with the connector A is illustrated. In Figure 2 the biomass is discharged through valve 10 from reactor vessel 4 and is passed to a manifold 12 from which it can be routed to a number of different individual piping systerns 14, which in Figure 2 are illustrated as spray irrigation lines with a plurality of spray heads 16 mounted at appropriate locations. It will be evident of course, that the line 14 can be any of a wide variety of types of irrigation devices, such as drip irrigation, spray irrigation, center pivot irrigation, or any mixture of types of systems. The manifold 12 can be either a simple manifold in which all of the lines 14 are supplied with liquid simultaneously, but more preferably will be either a manually or automatically controlled manifold in which the biomass slurry liquid is routed individually and separately to the different lines 14, depending on the particular irrigation needs of the soils and vegetation served by each of the lines 14. The sequencing can be automatic according to a predetermined program, can be manually operated or can be controlled in response to field conditions determined by sensors placed approximate to the various lines 14, so that the system responds as needed to the different soil and vegetation areas served by each of the lines on an as needed basis. For instance, if the system of Figure 2 were in use in a large park with varied terrain, those areas of grass which are open and subject to direct sunlight, or that vegetation which is growing in well drained soils, would most commonly receive the slurry liquid more frequently than other areas of the park which are shaded or where the vegetation is growing in highly water retentive soils.

If desired, the system of Figure 2 could be operated without a manifold 12 at all, so that the basic system of Figure 1 feeds directly into a line 14. This type of operation is likely to be used only in very limited circumstances, since it does not permit a variation for different application needs, such as different soil conditions in the area being irrigated.

Figure 3 illustrates another type of distribution system which is particularly useful for large open areas such as athletic fields, large parks or row or tree crops. In this case, the biomass slurry from tank 4 is passed to an accumulation or holding supply tank 18 from which it can be dispersed as needed through valve 20 to nozzle 22 from which it is flowed into tank 24 mounted on truck 26. Truck 26 is equipped with a spray system 28 from which the biomass liquid in tank 24 can be pumped and applied to the ground or to neighboring vegetation, including trees. Truck 26 can be driven to remote locations and there used to apply the biomass slurry to such things as row crops, orchard fruit trees or soil, or large open park or golf course areas for which pipeline irrigation systems such as that shown in Figure 2 are not practical. The system of Figure 3 is particularly useful where there are a large number of scattered plots to be treated with the biomass liquid and where a single tank truck 26 could move readily from plot to plot and carry enough biomass slurry liquid to irrigate and treat all of the plots on its route.

Yet another system is shown in Figure 4, which consists of a holding tank or supply tank 30 from which the liquid can be passed to a manifold 32 and from there into conduits 34, each of which leads to a satellite supply tank 36, each of which in turn provides slurry liquid through various types of irrigation treatment systems 38. Passage of the slurry from manifold 32 to individual conduits 34 is controlled by valves 40 which in turn are controlled by a controller 42. Controller 42, and also controller 44, which controls valve 20 in the system of Figure 3, can be a separate unit or can be part of the control system 6 of Figure 1. Similarly, if it is desired to pump the liquid slurry directly from tanks 4 into the systems of Figure 3 or Figure 4, tanks 18 and 30 (Figures 3 and 4, respectively) can be eliminated from the system. The presence of tanks 18 and 30 is preferred however, in that they allow for accumulation of the liquid slurry so that it can be dispersed as needed and so that the reactors 4 can be used for other purposes without disrupting the operations of the systems of Figures 3 and 4 for local operation.

Alternatively, in Figure 4, if the distances involved between the central system and the satellites 46 are excessive one or more of the conduits 34 of the system can be eliminated. In this case, the slurry can be loaded into a tank truck 48 and the truck driven to the satellite location where the slurry is transferred from the truck's tank to the tank 36.

Yet other means of dispersion are illustrated in Figure 12. Part A of Figure 12 shows dispersion from an aircraft 120. Tanks within the aircraft's body 122 are connected by pipes to spray heads 124 on the outside of the aircraft, from which the slurry is dispensed as spray 128 onto the desired turf, crops or other plants 126. Use of aircraft is of course particularly suited for application of the present compositions to large or remote areas. Similarly, in Part B of Figure 12, a small hand- or power-propelled device 130 similar in size to a lawn mower has a tank 132 mounted on it, with dispensers or spray heads 138 mounted underneath and connected to the tank 132 by suitable valved conduits. Handle 134, which may also contain the valve controls, is used to push or guide the device 130. This type of device is particularly suited to application of the compositions to small or delicate areas, illustrated here as a golf green 136.

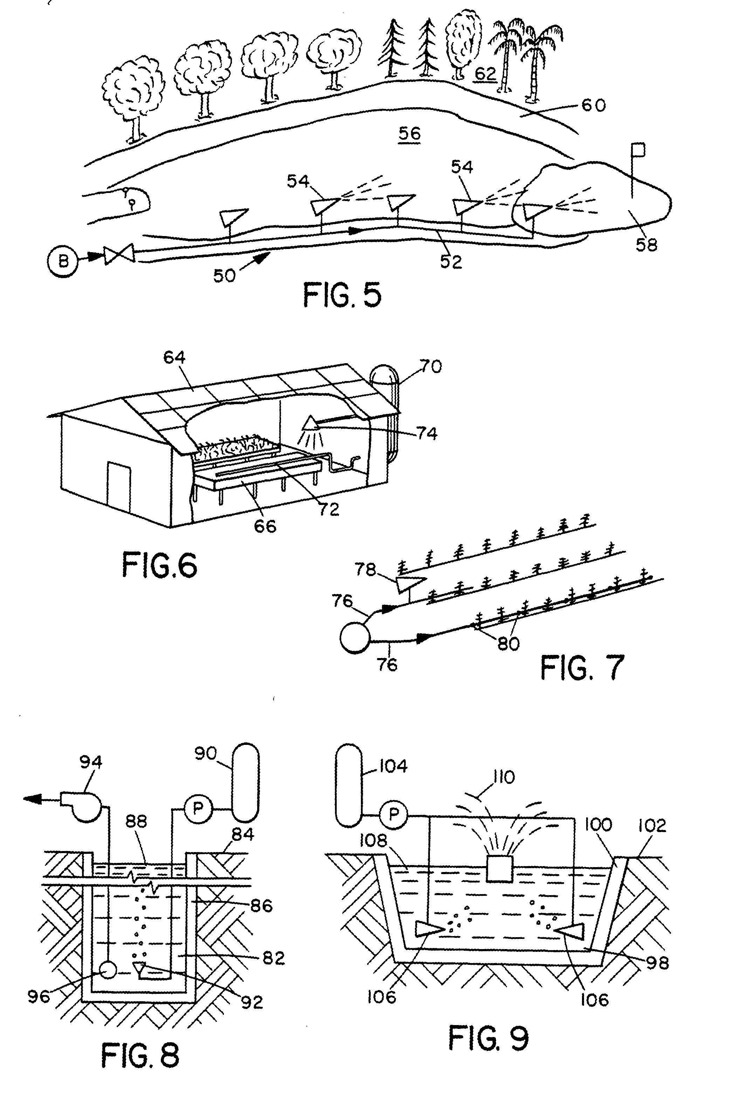

Various end uses of the present invention are shown in Figures 5-9. Figures 5-7 illustrate use for soil treating, vegetation growth and turf enhancement, while Figures 8 and 9 illustrate various forms of water treatment. In Figure 5, the irrigation system 50 is shown as connected to one or another of the systems in Figures 2 or 4, as illustrated at B. The liquid slurry is pumped through one or more conduits 52 to which are mounted various irrigation devices such as spray head 54 and from which the slurry is sprayed out over the various areas of turf or vegetation to be treated. In the situation illustrated in Figure 5, the area is a golf course and the turf areas being irrigated by the sprays from nozzles 54 are fairways 56 and greens 58. Of course as desired, and depending on the type of terrain and local climate, the various irrigation systems 52 can also be used to irrigate areas of rough 60 or other areas 62 of the course. It will be understood that there will be a plurality of conduits 52 and that they will be controlled by various manifolds and, if needed, pumps (not shown), all in the conventional manner for irrigation of golf courses, parks and similar areas. Similar results will be obtained by treatment of other vegetation areas such as parks, athletic fields, road and railroad rights-of-way borders, greenbelts, lawns and the like.

Figure 6 illustrates the use of the present system for horticulture as illustrated by greenhouse 64, shown partially cut away. Within the greenhouse are a variety of plant growing boxes or troughs 66, each of which is filled with soil and is used to grow the individual plants 68. This system of this invention can be enclosed in the housing 70 so that the greenhouse is operated directly with a single individual system. Alternatively, an individual system can be mounted in the housing 70 and used to supply not only greenhouse 64, but also neighboring greenhouses (not shown), since it is common in many commercial horticulture operations to have a number of greenhouses closely situated together, either for growing large numbers of the same types of plants or for growing a variety of different plants in the different greenhouses. Also alternatively, the housing 70 can encompass one of the satellite units 36 as illustrated in Figure 4.

Regardless of whether it is a primary or a satellite system which is in the housing 70, the biomass slurry is piped into the greenhouse and distributed to the plants in one or more of different conventional routes, including direct feed to the plant trough 66 as illustrated at 72, which may be a simple liquid feed system or a drip irrigation system, or overhead spray of the plants as illustrated at 74. Other routes for applying the slurries to the plants, such as handheld hoses and nozzles connected to the slurry system, may also be used. Those familiar with greenhouse horticulture will be aware of the numerous methods of applying the slurry to the plants or the bedding soil in the troughs 66, and all of these are equally suitable for use in the present invention.

Yet another end use application is illustrated in Figure 7, large area production agriculture. Again the particular irrigation systems 76 are shown as being connected to the system at B, and the variety of different irrigation units which can be used are illustrated by a spray nozzle 78 and drip irrigation nozzles 80. Figure 7 illustrates row crops such as corn, or beans, but it will also be readily understood that the present invention is also applicable to field crops such as wheat and orchard or tree crops such as fruit trees and various types of nuts. This system is also applicable to both linear irrigation systems and also center pivot and cross field movable irrigation systems.

Numerous desirable properties of soil can be enhanced by treating with the present process and the microorganisms from the original liquid feed, which are multiplied and dispersed over soil and vegetation areas. Nitrogen fixation in the soil is improved and increased, thus leading to enhanced crop or other vegetation growth. Nutrient availability in the soil for vegetation growth is improved, as is the ability of plant roots to access such nutrients. Dispersion of growth factors, regulators and metabolites into the soil may be accomplished, which in turn will enhance growth of vegetation which utilize such materials. Conversely, undesirable properties of the soil, which inhibit vegetation growth, can be reduced or eliminated. Treatment of the soil with microorganism-specific antibiotics can eliminate harmful pathogens which are initially present in the soil without adverse effect on desirable microorganisms supplied to the soil through the present process. Similarly, various harmful organic or inorganic compounds, such as salts, hydrocarbons and pesticide residues, may be eliminated or their effects reduced by action of specific microorganisms supplied herein. Thus, soil which has previously been capable of only low yield of crops, poor support of vegetation, or even devoid of sustainable vegetation can be remediated by treatment by the present process, to the point where lush, healthy vegetation and abundant crops can be grown, and then subsequently maintained by continued application of the present process. It will of course be understood that the quantities and types of microorganisms, nutrients, enzymes, metabolites and other materials supplied by the present process will vary depending on the types of soil and vegetation involved, whether the process is being used for remediation or maintenance of soil and vegetation, the local topography, whether edible crops are being grown, and the like. Those skilled in the art will have no difficulty determining the optimum types of microorganisms and other materials to provide through the initial liquid feed, the amount of biomass to produce, and the application of the biomass to the designated soil or vegetation.

Figures 8 and 9 illustrate various types of water treatment which can be accomplished by the present invention. In Figure 8 the system of the present invention is used to purify contaminated well water. A well 82 formed in the ground 84 and framed by a porous lining 86 is shown filled with water 88. The biomass slurry of the present invention is contained in tank 90 from which it is pumped to a sparger or other dispersing device 92 submerged in the well, preferably near the bottom. As the biomass disperses into the water and remains there for an appropriate period of time, the biomass and the microorganisms and enzymes gradually eliminate the various contaminants in the water, so that after a period of time (usually a matter of days or weeks) the well water is substantially decontaminated and can be used by being pumped out by pump 94 drawing water through inlet 96. If the contamination is entering the well as ground water in the soil 84 through the porous lining 86, the rate of withdrawal of the water, once the well water 88 is initially purified, should be maintained at a rate at or below the rate at which the biomass slurry being pumped in from tank 90 can decontaminate the incoming water.

In Figure 9, a waste water effluent pond 98 is formed with an impervious liner 100 sunk into soil 102. The biomass slurry is contained in tank 104 from which it is pumped to spargers or similar dispersing devices 106 which are submerged in the effluent body 108. The biomass is pumped into the water and replenished as needed over a period of time necessary to reduce the contamination and the effluent to the desired level. The biomass will effect a reduction of the solid content of the effluent, especially the organic solids, and will also serve to detoxify toxic contaminants in the effluent. It is contemplated that a single treating pond 98 may not be sufficient in many cases and the treated effluents will have to be transferred sequentially to additional ponds for further treating in order to obtain the overall degree of contaminants, solid and toxin reduction and elimination that is desired. Also, the treatment by the biomass of the present invention preferably will be conducted in conjunction with conventional effluent treating processes such as aeration 110 and filtration.

As with soil and vegetation enhancement or remediation, water treatment by the process herein produces substantial improvements in water quality. Injection of the biomass materials herein into a body of water, such as well water or water retained in a container such as a tank, can improve the water quality by elimination or reduction of organic materials and salts and by detoxification of toxins. The same will be true, on a greater scale, for treatment of heavily contaminated water supplies, such as wastewater effluents or contaminated ground water aquifers. The ground waters also benefit indirectly from the treatment of soils described above, since the presence of an active microbial region in the upper layers of soil will denitrify nitrates and immobilize ammoniacal nitrogen, thus preventing conversion to nitrates at a rate greater than such nitrates can be utilized by the local growing vegetation. Direct treatment of ground water is illustrated in Figure 13, in which an "active" layer 25 of soil is created in which the microorganisms, enzymes and nutrients of this invention are contained. Layer 25 is at the top of the soil profile, is commonly a few feet deep and usually includes ground surface 27. A pipe with a spray head 29 and suitable pumping means (not shown) reaches down to aquifer 31 and draws contaminated ground water from the aquifer 31 into the pipe through inlet 33. The water is sprayed out 37 onto the ground surface 27, from which it soaks back into the ground. It passes through active layer 25 where it is at least partially decontaminated. The decontaminated water continues to flow through deeper strata or soil layers 35 until it returns to the aquifer 31. Continued pumping, spraying and passage of the water through the active layer will eventually produce a substantial reduction in the contaminant level in the aquifer water.

Other applications of this process of water and/or soil treatment are also contemplated, though not specifically identified. For example, it is understood that septic tanks are particularly susceptible to decontamination by this process. The contaminated water of the septic tank may be removed, treated by the disclosed process and returned to the tank or the biomass may be delivered directly to the septic tank or some similar treatment may be implemented. Similarly, the soil to be treated by the disclosed process may be located essentially anywhere and used to grow virtually anything. For instance, it will be recognized that this process may be used to enhance soil which is used to graze animals or to enhance residential yards or gardens, as well as commercially used soils.

The microorganisms preferably useful in this invention grow by means of a consistent supply of nutrients and availability of adequate oxygen. It is not preferred to use microorganisms which rely on photosynthesis and are therefore dependent upon the presence of light for growth. However, if it is desired to use such microorganisms, the vessel 4 can incorporate its own lighting system to provide light for the biomass growth either by having vessel 4 be made of a clear or translucent material which allows ambient light into the biomass within or by having artificial light positioned such that the artificial light is focused into the biomass.

The growth of the microorganisms may also be aided by mechanical mixing of the aqueous biomass in the vessel, as by use of a mechanical mixer or by recycle of the aqueous biomass through an external recycle conduit (not shown). Both recycle and mixer usage are common in biomass vessels and those skilled in the art will be well aware of the techniques and equipment available and how such should be used. The degree of turbulence induced by either the mixer or the recycle must be kept within relatively low limits, to avoid shear degradation of the microorganism biomass, particularly as the microorganism biomass becomes concentrated toward the end of each growth cycle.

Typical of the various materials which can comprise the biomass are different microorganisms, either singly or in various mixtures in the aqueous suspensions, together with appropriate nutrients and enzymes. Many such microorganisms, nutrients and enzymes are commercially sold as proprietary products. Those skilled in the art are able to determine the materials appropriate for their needs by selecting those which provide the desired seed treating functions, such a growth enhancement, disease-resistance and/or pest-resistance. For example, *Azospirillum brasilense* is useful for non-symbiotic nitrogen fixation on grasses and for seedling establishment enhancement. *Rhizobium ssp.* are useful for symbiotic nitrogen fixation in legumes. The *Bacillus* species *licheniformis*, *subtilis* and *polymyxa,* on the other hand, are useful for general soil improvement such as aggregate formation and stabilization. Additionally, most species of the *Bacillus* genus are useful as hyper-cellulase producing organisms for thatch and/or rubble degradation. *Gliocladium spp.* provide control from diseases, such as root rot and the fungal disease, phytophthora, and *Trichoderma spp.* are useful both as a broad spectrum disease control microorganism and as a hyper-cellulase producing organism.

Examples of other useful microorganisms include thermophiles such as *Archaebacteria,* described in Brock et al., *Biology of Microorganisms* (5th edn., 1988) § 18.6; microorganisms which utilize hydrocarbons as nutrients, such as *Pseudomonas* and *Mycobacterium* (Brock et al., § 16.23); nitrogen fixating bacteria such as *Azotobacter spp.*, *Cyanobacteria* and *Bacillus* *polymyxa* (Brock et al., § 16.24); and halophiles such as *Halobacterium* (Brock et al., § 19.33). Additional typical microorganisms are oxygen-generating bacteria exemplified by a microorganism product commercially available under the trade name "AG-14" from Natural Oxygen Products of El Paso, Texas, and described in U.S. Patent No. 3,855,121. Similar microorganisms include *Pseudomonas*, *Flavobacterium*, *Euglina spp*. and the three *Bacillus* species previously discussed. See, e.g., Brock et al., §§ 19.15, 19.20, and 19.26 and Moore et al., *Biological Science* (1963), pp. 248-249.

Numerous microbial nutrients and enzymes are also known, such as those exemplified by a product commercially available under the trade name "BNB-931" from Westbridge Company of Carlsbad, California, and a chelated product commercially available under the trade name "Sun-Up". The chelating agent in "Sun-Up" is citric acid. Microorganisms which function as insecticides, fungicides, metabolites and/or herbicides may also be part of they biomass. A particularly preferred product useful in this regard is a fungicidal/nematocidal product in which the active ingredient is *Burkholderia cepacia*, type Wisconsin, in a concentration of 10⁵ cells/gram. This product is commercially available under the trade name "Deny" from CCT Corporation of California. Another useful product is "Azo-Kote" from Encore Technologies, Inc. of Minnesota, which has *Azospirillium brasilense* as its active ingredient and is useful for non-symbiotic nitrogen fixation.

The above are only examples of the various materials that may comprise the biornass and are in no way intended to limit the scope of the invention. It is intended to include, as part of the concept of this invention, both currently known and commercially available microorganisms, enzymes and nutrients and those of similar function which become available and approved for seed treating applications in the future.

It will be evident that there are numerous embodiments of this invention which, while not expressly described above, are clearly within the scope of the invention. The above description is therefore intended to be exemplary only, and the scope of the invention is to be limited solely by the appended claims.

## Claims

1. A method for remediation and enhancement of soil or water, the method comprising the steps of:
forming a concentrated aqueous suspension of micro-organisms and nutrients therefor together in a first vessel (2);
providing a plurality of such suspensions in a plurality of such vessels;
injecting said aqueous suspensions as batches into a substantially larger volume of water in a common second vessel (4);
retaining said larger volume of water with said suspension dispersed therein in said second vessel (4) at a temperature and for a time sufficient for said micro-organisms to feed on at least a portion of said nutrients, reproduce and multiply into a concentrated biomass (13) containing a remainder of said nutrients and an increased number of said micro-organisms in said water;
thereafter dispensing said biomass (13) from said second vessel (4) and dispersing said biomass (13) on soil or water; and
maintaining said micro-organisms alive and active with said remainder of said nutrients for a period of time sufficient to enhance predetermined desirable properties of said soil or water or reduce predetermined undesirable properties of said soil or water.

2. A method as in claim 1 where said aqueous suspension further comprises enzymes, vegetation growth factors, vegetation growth regulators, antibiotics or metabolites.

3. A method as in claim 1 wherein a first individual suspension in said plurality of concentrated aqueous suspensions comprises components different from components in a second individual suspension in said plurality.

4. A method as in claim 1 wherein said first and second suspensions in respective first vessels (2,2',2",2A,2B,2C,2D) are injected into said large volume of water in said second vessel (4,4') at different times during a biomass cycle.

5. A method as in claim 1 comprising:
providing a plurality of sets of first and second vessels (2,2'2,"4), each set comprising a plurality of first vessels (2,2',2") and a common second vessel (4), each of which sets contains a biomass in a stage of growth different from biomasses in other sets or which is empty of biomass and water and is undergoing preparation for water addition, subsequent aqueous suspension injection into said water, and resulting biomass growth.

6. A method as in claim 1 wherein a cycle of vessel preparation, addition of water, biomass growth and biomass dispensing exists for each set of vessels (2,2',2",4) in said plurality.

7. A method as in claim 1 wherein said micro-organisms include species having indeterminate growth.

8. A method as in claim 7 further comprising macerating a biomass including said species to comminute said biomass into portions adapted to flow in a liquid slurry.

9. A method as in claim 1 wherein said biomass is dispersed onto soil.

10. A method as in claim 9 wherein said soil supports turf, field crops, ornamental plants, row crops or tree crops.

11. A method as in claim 9 wherein said biomass is dispersed onto said soil in the form of an aqueous slurry of said biomass.

12. A method as in claim 11 wherein dispersion of said biomass onto said soil comprises transferring said biomass from said second vessel into a further tank and thereafter dispersing said biomass onto said soil from said tank.

13. A method as in claim 12 wherein said further tank is part of a mobile vehicle and said mobile vehicle transports said biomass from said second vessel to said soil.

14. A method as in claim 13 wherein said vehicle comprises an aircraft, truck, trailer or hand-propolled or guided mobile device and said dispersing comprises dispersing said biomass onto said soil from said vehicle.

15. A method as in claim 11 wherein dispersion of said biomass onto said soil comprises transporting said aqueous slurry of said biomass through a liquid, conduit from said second vessel to a spray nozzle and thereafter dispersing said biomass onto said soil from said spray nozzle.

16. Apparatus for remediation and enhancement of soil or water which comprises;
a plurality of first vessels (2,2',2") for receiving concentrated aqueous suspensions of micro-organisms and nutrients therefor;
a common second vessel (4) larger than each of said first vessels (2,2',2");
said first vessels (2,2',2") being provided within said second vessel (4);
a liquid conduit between said second vessel (4) and said plurality of first vessels (2,2'2");
an injector for moving said aqueous suspensions into a volume of water substantially greater than the volume of one of said first vessels (2,2'2") and within said second vessel (4);
operating means for producing and maintaining conditions over a period of time within said second vessel (4) conducive to reproduction and growth of said micro-organisms in the presence of said nutrients, such that said micro-organisms during said period of time multiply into an aqueous biomass comprising said micro-organisms dispersed in the vessel water; and
dispensing means (3,10) for removing aqueous biomass from said second vessel (4);
such that said aqueous biomass can subsequently be dispersed into soil or water and said micro-organisms therein maintained alive and active for a period of time sufficient to enhance predetermined desirable properties of said soil or water or reduce predetermined undesirable properties of said soil or water.

17. Apparatus as in claim 16 wherein each of said plurality of first vessels (2,2',2") is provided with an individual injector and conduit to said second vessel (4).

18. Apparatus as in claim 17 further comprising a controller (6) to regulate rate and timing of injection of said aqueous suspensions from each respective first vessel (2,2'2") into said second vessel (4).

19. Apparatus as in claim 18 further comprising a plurality of sets of said first and second vessels (2,2',2",4), each adapted to permit growth of a micro-organism biomass in isolation from micro-organism biomasses in other sets of vessels in said plurality.

20. Apparatus as in claim 19 wherein said set of vessels in said plurality of sets are housed in a single housing (4').

21. Apparatus as in claim 16 further comprising liquid conveyance means to transport said dispensed aqueous biomass from said second vessel to said soil or water.

22. Apparatus as in claim 21 wherein said conveyance means comprises a liquid conduit (14) and a spray nozzle (16) to spray said aqueous biomass onto said soil or water.

23. Apparatus as in claim 21 wherein said conveyance means comprises a vehicle having a tank (24) into which said aqueous biomass is loaded for transport to said soil or water.

24. Apparatus as in claim 23 wherein said aqueous biomass is dispersed directly from said tank (24) to said soil or water.

25. Apparatus as in claim 23 wherein said vehicle comprises an aircraft (120), truck (26), trailer, hand-propelled or guided mobile device.

26. Apparatus as in claim 16 wherein said biomass is continuously dispensed from said vessel (4) while said vessel (4) is continuously supplied with said aqueous suspension; such that the volume of said vessel (4) is not depleted.

## Patentansprüche

1. Verfahren zum Entgiften von Boden oder Wasser, mit folgenden Schritten:
Bildung einer konzentrierten wässrigen Suspension aus Mikroorganismen und Nährstoffen für diese in einem ersten Behälter (2);
Herstellen einer Mehrzahl derartiger Suspensionen in einer Mehrzahl derartiger Behälter;
Injizieren der wässrigen Suspensionen als Chargen in wesentlich größeres Volumen von Wasser in einem gemeinsamen zweiten Behälter (4);
Halten des großeren Volumens von Wasser mit der darin dispergierten Suspension in dem zweiten Behälter (4) bei einer Temperatur und über eine Zeit, die ausreicht, daß die Mikroorganismen wenigstens einen Teil der Nährstoffe fressen, sich reproduzieren und vermehren zu einer konzentrierten Biomasse (13), die einen Rest der Nährstoffe und eine vergrößerte Anzahl von Mikroorganismen in dem Wasser enthält;
Abgeben der Biomasse (13) aus dem zweiten Behälter (4) und Dispergieren der Biomasse (13) auf Boden oder Wasser; und
Erhalten der Mikroorganismen lebend und aktiv mit dem Rest der Nährstoffe über eine Zeit, die ausreicht, vorbestimmte wünschenswerte Eigenschaften des Bodens oder Wassers zu fördern oder vorbestimmte unerwünschte Eigenschaften des Bodens oder Wassers zu reduzieren.

2. Verfahren nach Anspruch 1, bei dem die wässrige Suspension weiterhin Enzyme, Vegitationswachstumsfaktoren, Vegitationswachstumsregulatoren, antibiotische Stoffe oder Stoffwechselstoffe enthält.

3. Verfahren nach Anspruch 1, bei dem eine erste individuelle Suspension in der Mehrzahl der konzentrierten wässrigen Suspensionen Komponenten enthält, die sich von Komponenten in einer zweiten individuellen Suspension der Mehrzahl unterscheiden.

4. Verfahren nach Anspruch 1, bei dem die ersten und zweiten Suspensionen in den ersten Behältern (2,2',2'',2A,2B,2C,2D) in ein großes Volumen von Wasser in dem zweiten Behälter (4,4') zu unterschiedlichen Zeiten während eines Biomasse-Zyklus injiziert werden.

5. Verfahren gemäß Anspruch 1, bei dem man eine Anzahl von Sätzen von ersten und zweiten Behältern (2,2',2'',4) bereitstellt, von denen jeder Satz eine Mehrzahl von ersten Behältern (2,2',2'') und einen gemeinsamen zweiten Behälter (4) umfaßt und bei dem jeder Satz eine Biomasse in einem anderen Wachstumsstadium enthält, als es in den anderen Sätzen der Fall ist oder bei dem Behälter keine Biomasse und Wasser enthalten und für die Wasserzugabe vorbereitet werden und anschließend eine wässrige Suspension in das Wasser injiziert wird und die Biomasse wächst.

6. Verfahren nach Anspruch 1, bei dem ein Zyklus der Vorbereitung der Behälter, der Zugabe von Wasser, des Wachstums der Biomasse und der Abgabe der Biomasse für jeden der Behältersätze (2,2',2'',4) der Mehrzahl der Behältersätze stattfindet.

7. Verfahren nach Anspruch 1, bei dem die Mikroorganismen Spezies enthalten, die ein unbestimmtes Wachstum aufweisen.

8. Verfahren nach Anspruch 7, bei dem eine Biomasse, die die Spezies enthält, mazeriert wird, so daß die Biomasse in Portionen zerkleinert wird, die in einem flüssigen Schlamm fließen.

9. Verfahren nach Anspruch 1, bei dem die Biomasse auf den Boden dispergiert wird.

10. Verfahren nach Anspruch 9, bei dem der Boden Torf, Feldgetreide, Zierpflanzen, Reihengetreide oder Baumschnitt trägt.

11. Verfahren nach Anspruch 9, bei dem die Biomasse auf den Boden in der Form eines wässrigen Schlammes der Biomasse dispergiert wird.

12. Verfahren nach Anspruch 11, bei dem die Dispersion der Biomasse auf den Boden das Übertragen der Biomasse von dem zweiten Behälter in einen weiteren Tank und das anschließende Dispergieren der Biomasse auf den Boden von dem Tank umfaßt.

13. Verfahren nach Anspruch 12, bei dem der weitere Tank ein Teil eines beweglichen Fahrzeugs ist und das bewegliche Fahrzeug die Biomasse von dem zweiten Behälter auf den Boden überführt.

14. Verfahren nach Anspruch 13, bei dem das Fahrzeug ein Flugzeug, ein Lastwagen, ein Anhänger oder eine von Hand geschobene oder geführte bewegliche Einrichtung ist und das Dispergieren der Biomasse auf den Boden von dem Fahrzeug aus erfolgt.

15. Verfahren nach Anspruch 11, bei dem die Dispersion der Biomasse auf den Boden das Transportieren des wässrigen Schlammes der Biomasse durch eine Flüssigkeit in einer Leitung von dem zweiten Behälter zu einer Sprühdüse und anschließendes Dispergieren der Biomasse auf den Boden von der Sprühdüse aus umfaßt.

16. Vorrichtung zur Entgiftung von Boden oder Wasser, mit
einer Mehrzahl von ersten Behältern (2,2',2'') zur Aufnahme konzentrierter wässriger Suspensionen von Mikroorganismen und Nährstoffen für diese;
einem gemeinsamen zweiten Behälter (4), der größer ist als die ersten Behälter (2,2',2'');
welche ersten Behälter (2,2',2'') innerhalb des zweiten Behälters (4) angeordnet sind;
eine Flüssigkeitsleitung zwischen dem zweiten Behälter (4) und der Mehrzahl der ersten Behälter (2,2',2");
einem Injektor zum Bewegen der wässrigen Suspensionen in ein Wasservolumen, das erheblich größer ist als das Volumen eines der ersten Behälter (2,2',2") innerhalb des zweiten Behälters (4);
Betätigungseinrichtungen zum Erzeugen und Erhalten von Bedingungen über einen Zeitraum innerhalb des zweiten Behälters (4), die zur Reproduktion und zum Wachstum der Mikroorganismen in Anwesenheit der Nährstoffe führen, so daß die Mikroorganismen während der Zeitperiode sich zu einer wässrigen Biomasse vermehren, die die Mikroorganismen innerhalb des Behälterwassers enthält; und
Abgabeeinrichtung (3,10) zum Entfernen der wässrigen Biomasse von dem zweiten Behälter (4);
derart, daß die wässrige Biomasse anschließend auf den Boden oder Wasser abgegeben werden kann und die Mikroorganismen in der Biomasse über einen Zeitraum lebend und aktiv bleiben, der ausreicht zur Verbesserung vorbestimmter wünschenswerter Eigenschaften des Bodens oder Wassers und zur Reduzierung vorgegebener unerwünschter Eigenschaften des Bodens oder Wassers.

17. Vorrichtung nach Anspruch 16, bei der jede Mehrzahl der ersten Behälter (2,2',2'') mit einem eigenen Injektor und einer Leitung zu dem zweiten Behälter (4) versehen ist.

18. Vorrichtung nach Anspruch 17, mit einer Steuerung (6) zur Regelung der Menge und der Zeit, der Injektion der wässrigen Suspensionen von jedem der ersten Behälter (2,2',2") in den zweiten Behälter (4).

19. Vorrichtung nach Anspruch 18, mit einer Anzahl von Sätzen der ersten und zweiten Behälter (2,2',2'',4), die das Wachstum einer Mikroorganismen-Biomasse in Isolierung von den Mikroorganismen-Biomassen in anderen Sätzen der Behälter der Mehrzahl der Sätze gestatten.

20. Vorrichtung nach Anspruch 19, bei der die Sätze der Behälter in der Mehrzahl der Sätze innerhalb eines einzigen Gehäuses (4') untergebracht sind.

21. Vorrichtung nach Anspruch 16, mit Flüssigkeitsfördereinrichtungen zum Transportieren der abgegebenen wässrigen Biomasse aus dem zweiten Behälter auf den Boden oder das Wasser.

22. Vorrichtung nach Anspruch 1, bei der die Fördereinrichtung eine Flüssigkeitsleitung (14) und eine Sprühdüse (16) umfaßt, durch die die wässrige Biomasse auf den Boden oder auf Wasser gesprüht wird.

23. Vorrichtung nach Anspruch 21, bei dem die Fördereinrichtungen ein Fahrzeug umfassen, das einen Tank (24) aufweist, in den die wässrige Biomasse zum Transport zu dem Boden oder Wasser eingefüllt wird.

24. Vorrichtung nach Anspruch 23, bei der die wässrige Biomasse direkt von dem Tank (24) auf den Boden oder Wasser abgegeben wird.

25. Vorrichtung nach Anspruch 23, bei dem das Fahrzeug ein Flugzeug (120), ein Lastwagen (26), ein Anhänger oder eine von Hand geschobene oder geführte Einrichtung ist.

26. Vorrichtung nach Anspruch 16, bei der die Biomasse kontinuierlich von dem Behälter (4) abgegeben wird, während der Behälter (4) kontinuierlich mit der wässrigen Suspension gefüllt wird, derart, daß das Volumen des Behälters (4) nicht abfällt.

## Revendications

1. Procédé pour traiter et améliorer de la terre ou de l'eau, le procédé comportant les étapes consistant à :
former une suspension aqueuse concentrée de micro-organismes et de substances nutritives destinées à ceux-ci, ensemble dans un premier récipient (2),
disposer une pluralité de ces suspensions dans une pluralité de ces récipients,
injecter lesdites suspensions aqueuses sous forme de lots dans un volume nettement plus grand d'eau dans un second récipient commun (4),
conserver ledit plus grand volume d'eau avec ladite suspension dispersée à l'intérieur dans ledit second récipient (4) à une température suffisante et pendant un temps suffisant pour que lesdits micro-organismes s'alimentent sur au moins une partie desdites substances nutritives, se reproduisent et se multiplient dans une biomasse concentrée (13) contenant le reste desdites substances nutritives et un nombre accru desdits micro-organismes dans ladite eau,
après ceci, distribuer ladite biomasse (13) depuis ledit second récipient (4) et disperser ladite biomasse (13) sur de la terre ou de l'eau, et
maintenir les micro-organismes en vie et actifs avec ledit reste desdites substances nutritives pendant une période de temps suffisante pour améliorer des propriétés souhaitables prédéterminées de ladite terre ou eau ou réduire des propriétés indésirables prédéterminées de ladite terre ou eau.

2. Procédé selon la revendication 1, dans lequel ladite suspension aqueuse comporte de plus des enzymes, des facteurs de croissance végétative, des régulateurs de croissance végétative, des anticorps et des métabolites.

3. Procédé selon la revendication 1, dans lequel une première suspension individuelle parmi ladite pluralité de suspensions aqueuses concentrées comporte des composants différents des composants d'une seconde suspension individuelle de ladite pluralité.

4. Procédé selon la revendication 1, dans lequel lesdites première et seconde suspensions dans des premiers récipients respectifs (2, 2', 2", 2A, 2B, 2C, 2D) sont injectées dans ledit plus grand volume d'eau dans ledit second récipient (4, 4') à différents instants pendant un cycle de biomasse.

5. Procédé selon la revendication 1, comportant :
la fourniture d'une pluralité d'ensembles de premiers et second récipients (2, 2', 2", 4), chaque ensemble comportant une pluralité de premiers récipients (2, 2', 2'') et un second récipient commun (4), chacun des ensembles contenant une biomasse à un stade de croissance différent des biomasses des autres ensembles ou chacun étant vide de biomasse et d'eau et subissant une préparation pour un ajout d'eau, une injection de suspension aqueuse ultérieure dans ladite eau, et une croissance de biomasse résultante.

6. Procédé selon la revendication 1, dans lequel un cycle de préparation de récipient, d'ajout d'eau, de croissance de biomasse et de distribution de biomasse existe pour chaque ensemble de récipients (2, 2', 2", 4) de ladite pluralité.

7. Procédé selon la revendication 1, dans lequel lesdits micro-organismes incluent une espèce ayant une croissance indéterminée.

8. Procédé selon la revendication 7, comportant de plus la macération d'une biomasse incluant ladite espèce pour fragmenter ladite biomasse en parties adaptées à s'écouler sous forme d'une boue liquide.

9. Procédé selon la revendication 1, dans lequel ladite biomasse est dispersée sur de la terre.

10. Procédé selon la revendication 9, dans lequel la terre supporte du gazon, des cultures agricoles, de plantes ornementales, des cultures potagères ou des cultures arboricoles.

11. Procédé selon la revendication 9, dans lequel ladite biomasse est dispersée sur ladite terre sous la forme d'une boue aqueuse de ladite biomasse.

12. Procédé selon la revendication 11, dans lequel la dispersion de ladite biomasse sur ladite terre comporte le transfert de ladite biomasse dudit second récipient dans un réservoir supplémentaire et, après ceci, la dispersion de ladite biomasse sur ladite terre depuis ledit réservoir.

13. Procédé selon la revendication 12, dans lequel ledit réservoir supplémentaire est une partie d'un véhicule mobile, lequel véhicule mobile transporte ladite biomasse dudit second récipient à ladite terre.

14. Procédé selon la revendication 13, dans lequel ledit véhicule comporte un avion, un camion, une remorque ou un dispositif mobile guidé ou propulsé à la main et ladite dispersion comporte la dispersion de ladite biomasse sur ladite terre depuis ledit véhicule.

15. Procédé selon la revendication 11, dans lequel la dispersion de ladite biomasse sur ladite terre comporte le transport de ladite boue aqueuse de ladite biomasse à travers une conduite de liquide dudit second récipient à une buse de pulvérisation et, après ceci, la dispersion de ladite biomasse sur ladite terre depuis ladite buse de pulvérisation.

16. Dispositif pour traiter et améliorer de la terre ou de l'eau qui comporte :
une pluralité de premiers récipients (2, 2', 2") pour recevoir des suspensions aqueuses concentrées de micro-organismes et de substances nutritives destinées à ceux-ci,
un second récipient commun (4) plus grand que chacun desdits premiers récipients (2, 2', 2"),
lesdits premiers récipients (2, 2', 2") étant agencés dans ledit second récipient (4),
une conduite de liquide entre ledit second récipient (4) et ladite pluralité de premiers récipients (2, 2', 2"),
un injecteur pour déplacer lesdites suspensions aqueuses dans un volume d'eau nettement plus grand que le volume d'un desdits premiers récipients (2, 2', 2") et situé au sein dudit second récipient (4),
des moyens opérationnels pour produire et maintenir des conditions sur une période de temps dans ledit second récipient (4) permettant une reproduction et une croissance desdits micro-organismes en présence desdites substances nutritives, de sorte que lesdits micro-organismes pendant ladite période de temps se multiplient dans une biomasse aqueuse comportant lesdits micro-organismes dispersés dans l'eau du récipient, et
des moyens de distribution (3, 10) pour retirer la biomasse aqueuse dudit second récipient (4),
de sorte que ladite biomasse aqueuse peut par la suite être dispersée sur la terre ou l'eau et lesdits micro-organismes à l'intérieur sont maintenus en vie et actifs pendant une période de temps suffisante pour améliorer des propriétés souhaitables prédéterminées de ladite terre ou eau ou réduire des propriétés indésirables prédéterminées de ladite terre ou eau.

17. Dispositif selon la revendication 16, dans lequel chaque récipient de ladite pluralité de premiers récipients (2, 2', 2") est muni d'un injecteur individuel et d'une conduite vers ledit second récipient (4).

18. Dispositif selon la revendication 17, comportant de plus un contrôleur (6) pour réguler le débit et le cadencement d'injection desdites suspensions aqueuses depuis chaque premier récipient respectif (2, 2', 2") dans ledit second récipient (4).

19. Dispositif selon la revendication 18, comportant de plus une pluralité d'ensembles desdits premiers et second récipients (2, 2', 2", 4), chacun adapté pour permettre une croissance d'une biomasse de micro-organismes en étant isolé des biomasses de micro-organismes des autres ensembles de récipients de ladite pluralité.

20. Dispositif selon la revendication 19, dans lequel ledit ensemble de récipients de ladite pluralité d'ensembles est enfermé dans une enceinte unique (4').

21. Dispositif selon la revendication 16, comportant de plus des moyens d'acheminement de liquide pour transporter ladite biomasse aqueuse distribuée depuis ledit second récipient vers ladite terre ou eau.

22. Dispositif selon la revendication 21, dans lequel lesdits moyens d'acheminement comportent une conduite de liquide (14) et une buse de pulvérisation (16) pour pulvériser ladite biomasse aqueuse sur ladite terre ou eau.

23. Dispositif selon la revendication 21, dans lequel lesdits moyens d'acheminement comportent un véhicule ayant un réservoir (24) dans lequel ladite biomasse aqueuse est chargée pour le transport sur ladite terre ou eau.

24. Dispositif selon la revendication 23, dans lequel ladite biomasse aqueuse est dispersée directement depuis ledit réservoir (24) sur ladite terre ou eau.

25. Dispositif selon la revendication 23, dans lequel ledit véhicule comporte un avion (120), un camion (26), une remorque, un dispositif mobile guidé ou propulsé à la main.

26. Dispositif selon la revendication 16, dans lequel ladite biomasse est distribuée en continu depuis ledit récipient (4) alors que ledit récipient (4) est alimenté en continu par ladite suspension aqueuse, de sorte que le volume dudit récipient (4) n'est pas épuisé.
